# EUROPÄISCHE PATENTSCHRIFT

(11) **EP 0 638 283 B1**
(45) Veröffentlichungstag und Bekanntmachung des Hinweises auf die Patenterteilung: **03.06.1998**
(21) Anmeldenummer: 94112304.4
(22) Anmeldetag: 06.08.1994
(51) Int. Cl.: A61B 5/087

(54) **Instrument zum Messen der maximalen Strömungsmenge während einer Ausatmung**
Maximum expiratory flow measuring device
Appareil pour mesurer le débit expiratoire de pointe

(30) Priorität: 14.08.1993 DE 4327446
(43) Veröffentlichungstag der Anmeldung: 15.02.1995
(73) Patentinhaber: Carvalho da Silva, José, 97659 Schönau (DE)
(72) Erfinder: Carvalho da Silva, José, 97659 Schönau (DE)
(74) Vertreter: Fuchs Mehler Weiss

(56) Entgegenhaltungen:
- WO-A-92/21163
- WO-A-93/06778

## Beschreibung

Die Erfindung geht aus von einem Instrument zum Messen der maximalen Strömungsmenge während einer Ausatmung, gemäß dem Oberbegriff des Patentanspruchs 1.

Derartige Instrumente, die als "Peak-flow-meter" bezeichnet werden, sind an sich bekannt und werden zur Therapiekontrolle der Lungenfunktion, bei der die expiratorische Ausatmungsspitzengeschwindigkeit eine wesentliche Rolle spielt, in Zusammenarbeit mit dem behandelnden Arzt verwendet. So ist beispielsweise aus der DE 24 33 994 A1 ein Instrument der eingangs beschriebenen Art bekannt, bei dem die im Gehäuse angeordnete Stange mit einem Ende nicht lösbar in der Gehäusewand befestigt ist und auf dem anderen Ende die auf die Lufteinlaßöffnung gesteckte Aufnahme für ein Mundstück aufgeschraubt ist. Aus der US-4,944,306 ist ein Instrument der eingangs erwähnten Art bekannt, bei dem die Gehäusehälften und die Aufnahme für ein Mundsrück fest miteinander verschweißt sind und die Stange unlösbar innerhalb des Gehäuses befestigt ist. Weiterhin sind Instrumente der eingangs erwähnten Art bekannt, bei denen beispielsweise die Gehäusehälften miteinander verschraubt sind und die Aufnahme für das Mundstück mittels manuell nicht zu lösende Arretierungen auf der Einlaßöffnung befestigt ist.

Alle diese bekannten Instrumente sind aufgrund ihrer Konstruktionsweise nur schwer und umständlich, zum Teil sogar nur mit Werkzeug zu montieren. Alle diese bekannten Geräte lassen sich weiterhin, ebenfalls aufgrund ihrer Konstruktionsweise, nur schwer bzw. überhaupt nicht reinigen, da sie gar nicht oder nur mit Werkzeug äußerst umständlich demontierbar sind. Eine vollständige Demontage der Instrumente ist aber zum gründlichen und vollständigen Reinigen derselben absolut notwendig, da durch die warme und feuchte in das Gerät eingeblasene Ausatmenluft äußerst günstige Voraussetzungen für das Anwachsen von Bakterien und Pilzen innerhalb der Instrumente geschaffen werden und diese nur durch gründliches reinigen der Einzelteile entfernt werden können.

Aufgabe der vorliegenden Erfindung ist es daher, ein Instrument der eingangs erwähnten Art so weiterzuentwickeln, daß es einfach und kostengünstig manuell ohne Werkzeug zu montieren und demontieren ist und damit die Voraussetzungen für ein vollständiges Reinigen des Instrumentes und eine hygienische Anwendung bietet.

Diese Aufgabe wird mit dem Gegenstand des Anspruchs 1 gelöst. Die Unteransprüche geben vorteilhafte Ausgestaltungen des Instrumentes an.

Eine leichte Montage und Demontage des Instrumentes wird dadurch erzielt, daß eine erste Gehäusehälfte mindestens einen Vorsprung aufweist, der bei zusammengefügtem Gehäuse in eine Ausnehmung in der zweiten Gehäusehälfte eingreift, daß die erste und die zweite Gehäusehälfte jeweils mindestens einen Fortsatz aufweisen, die bei zusammengefügtem Gehäuse aneinander anliegen und daß die beiden Fortsätze bei zusammengefügtem Gehäuse durch ein darüber geschobenes Halteelement lösbar aneinander gehalten werden.

Durch diese konstruktive Ausgestaltung des Gehäuses ist eine einfache und schnelle Montage des Instrumentes ohne Werkzeug möglich, so daß ein erheblicher Kostenvorteil bei der Herstellung des Instrumentes erzielt wird. Nach Gebrauch des Instrumentes ist dieses, aufgrund dieser konstruktiven Gestaltung des Gehäuses, einfach manuell ohne großen Kraftaufwand zu demontieren und nach einer durchgeführten Reinigung wieder problemlos zusammenzufügen, so daß diese auch von alten oder kranken Menschen und Kindern vorgenommen werden kann, und die Voraussetzungen für eine hygienische Anwendung des Instrumentes gegeben sind.

Die Fortsätze sind an den jeweiligen Gehäusehälften vorteilhaferweise so ausgebildet, daß sie bei zusammengefügtem Gehäuse die Lufteinlaßöffnung umschließen. In einer bevorzugten Ausführungsform ist die Aufnahme für das Mundstück als Halteelement zum Aneinanderhalten der beiden Fortsätze ausgebildet und wird auf die die Lufteinlaßöffnung umschließenden Fortsätze aufgeschoben. Durch diese Ausbildung der Aufnahme erfüllt diese zwei Aufgaben, wodurch ein zusätzliches Bauteil eingespart wird, die Montage und Demontage aufgrund der reduzierten Teilezahl noch einfacher wird und bei der Herstellung des Instrumentes ein weiterer Kostenvorteil erzielt wird. Die Aufnahme für das Mundstück besteht in einer besonders bevorzugten Ausführungsform aus einem Hohlkörper, der an den beiden sich gegenüberliegenden Stirnseiten jeweils eine Öffnung aufweist. In die erste Öffnung wird das Mundstück eingeschoben und wird beispielsweise mittels bekannter selbsthemmender Einführschrägen in der Aufnahme gehalten, so daß ein einfaches und problemloses Auswechseln des Mundstückes möglich ist. In die zweite Öffnung werden die aneinander anliegenden Fortsätze der beiden Gehäusehälften eingeschoben. Durch diese spezielle Ausbildung der Aufnahme für das Mundstück werden die aneinander anliegenden Fortsätze vollständig umschlossen und somit bei Gebrauch fest und sicher aneinander gehalten.

Vorteilhafterweise wird die Aufnahme für das Mundstück mit einem Sicherungselement lösbar auf den beiden Fortsätzen arretiert, um ein unbeabsichtigtes Abziehen der als Halteelement ausgebildeten Aufnahme für ein Mundstück und damit ein unbeabsichtigtes Öffnen des Gehäuses zu verhindern In einer besonderen Ausführungsform ist dieses Sicherungselement klammerförmig ausgebildet und weist an den beiden Schenkeln nach innen überstehende Stege auf, die mit Nasen über die Enden der Schenkel hinausragen. Weiterhin ist an diesen nach innen überstehenden Stegen jeweils mindestens ein weiter nach innen hervorragender Vorsprung angeordnet. Vorteilhafterweise weist die aus einem Hohlkörper bestehende Aufnahme für ein Mundstück in der Mantelfläche zwei sich gegenüberliegende Öffnungen auf, die innerhalb von Vertiefungen angeordnet sind, wobei in die Vertiefungen die Schenkel des klammerförmigen Sicherungselementes eingeschoben werden. Dabei werden die Stege des Sicherungselementes durch die Öffnungen in den Vertiefungen der Mantelfläche hindurch in vorteilhafter Weise auf den Fortsätzen der beiden Gehäusehälften angeordnete Nuten eingeschoben. Zur Arretierung wird das klammerförmige Sicherungselement so weit in die Vertiefungen auf der Mantelfläche des Halteelementes eingeschoben, bis die Nasen an den Enden der Stege die Mantelfläche des Halteelementes von innen her hintergreifen und die an den Stegen angeordneten Vorsprünge in die vorteilhafterweise in den Nuten der Fortsätze angeordneten Ausnehmungen einrasten. Auf diese Weise wird ein lösbar arretierter Formschluß erzeugt, der manuell ohne großen Kraftaufwand zu lösen und wieder zusammenzufügen ist. So entsteht auf der einen Seite - durch den Verzicht auf Werkzeug bei Montage und Demontage - ein schon angesprochener Kostenvorteil bei der Herstellung, auf der anderen Seite ist die Durchführung der ebenfalls schon angesprochenen Reinigung zur Gewährleistung einer hygienischen Anwendung möglich.

Der an einer ersten Gehäusehälfte angebrachte Vorsprung, welcher bei zusammengefügtem Gehäuse in einer Ausnehmung in der zweiten Gehäusehälfte eingreift, ist vorteilhafterweise als eine hervorstehende Verriegelungsnase ausgebildet und greift in eine in der zweiten Gehäusehälfte als Luftauslaßöffnung vorgesehene Ausnehmung.

Durch diese spezielle Ausbildung der Verriegelungsnase in Verbindung mit den durch ein Halteelement aneinandergehaltenen Fortsätzen, welches durch ein Sicherungselement lösbar auf den beiden Fortsätzen arretiert ist, ist eine einfache und leichte Montage und Demontage des Gehäuses möglich. Zur Montage wird die an einer ersten Gehäusehälfte angeordnete Verriegelungsnase in die auf der zweiten Gehäusehälfte angeordnete Verriegelungsöffnung eingeschwenkt, so daß die beiden Gehäusehälften zusammengefügt werden und die beiden Fortsätze aneinander anliegen. Anschließend wird das Halteelement auf die beiden Forsätze aufgeschoben und mit dem Sicherungselement lösbar arretiert. Zur Demontage wird das Sicherungselement von dem Halteelement gelöst, das Halteelement von den beiden aneinanderliegenden Fortsätzen entfernt und anschließend die beiden Gehäusehälften auseinandergeschwenkt, so daß die Verriegelungsnase aus der Verriegelungsöffnung gleitet.

Zur Gewährleistung der leichten und einfache Montage und Demontage der im Gehäuse angeordneten Stange, der Prallplatte und der zwischen Gehäuse und Prallplatte angeordneten Federmitteln, welche für eine vollständige Reinigung aller Bauteile des Instrumentes notwendig ist, bestehen die Federmittel vorteilhafterweise aus einer Schraubenzugfeder, die um die im Gehäuse angeordnete Stange herum angeordnet ist. Besonders vorteilhaft weist diese Schraubenzugfeder an einem Ende einen in den Innenraum der Schraubenfeder gerichteten und die Mittelachse der Schraubenfeder schneidenden Schenkel auf, der mit einem Ende der Stange zwischen dieser und einem in der Lufteinlaßöffnung angeordneten Diffusor festgeklemmt wird.

In einer besonders bevorzugten Ausführungsform wird das andere Ende der Schraubenfeder auf die Prallplatte aufgeschraubt. Diese weist dazu vorteilhafterweise einen senkrecht zur Oberfläche stehenden durchbohrten Zapfen auf, durch den die Stange hindurch geschoben wird und auf den das Ende der Schraubenfeder aufgeschoben wird. Weiterhin ist vorteilhafterweise neben dem Zapfen eine senkrecht durch die Prallplatte hindurchführende Bohrung angeordnet, an der ein einmal um den Zapfen herumführender, auf der Plattenoberfläche angeordneter Gewindegang mit einer Steigung entsprechend der Steigung der Schraubenfeder beginnt. Zur Befestigung der Schraubenfeder an der Prallplatte wird die Schraubenfeder mit einem Ende auf den durchbohrten Zapfen geschoben, der vorteilhafterweise eine konische Außenform besitzt, bis das Ende der Schraubenfeder auf dem auf der Plattenoberfläche ausgebildeten Gewindegang zu liegen kommt. Anschließend wird die Schraubenfeder gedreht, so daß das Ende des die Schraubenfeder bildenden Drahtes auf dem Gewindegang entlang gleitet, bis es zur durch die Plattenoberfläche hindurchführenden Bohrung gelangt, welche in dem Gewindegang angeordnet ist, und durch weiteres Drehen durch die Bohrung hindurch auf die Rückseite des Gewindeganges gelangt. Bei weiterem Drehen der Schraubenfeder können nun beliebig viele Drahtwicklungen des die Schraubenfeder bildenden Drahtes auf die Rückseite der Prallplatte gebracht werden, so daß die Länge des Teils der Schraubenfeder, der zwischen Prallplatte und Diffusor angeordnet ist, genau eingestellt werden kann.

Auf diese Weise ist zum einen eine einfache und schnelle Montage und Demontage von Schraubenfeder, Prallplatte und Stange möglich, auf der anderen Seite können Schraubenfedern mit unterschiedlichen Längen, beispielsweise hervorgerufen durch Fertigungstoleranzen, auf eine vorgegebene Länge eingestellt werden. Auch sind Schraubenfedern mit unterschiedlicher Federcharakteristik zu verwenden, die durch dieses beschriebene Aufschrauben auf die Prallplatte auf die benötigte Federkraft eingestellt werden können.

Ein bevorzugtes Ausführungsbeispiel der Erfindung wird anhand der Figuren näher beschrieben. Es zeigen.
- Fig. 1: eine perspektivische Ansicht des Instrumentes zum Messen der maximalen Strömungsmenge während einer Ausatmung,
- Fig. 1A: eine perspektivische Ansicht der beiden zusammengefügten Gehäusehälften des Instrumentes, teilweise weggebrochen
- Fig. 2: eine perspektivische Ansicht der oberen Gehäusehälfte des Instrumentes,
- Fig. 3: eine perspektivische Ansicht der unteren Gehäusehälfte des Instrumentes,
- Fig. 4: eine perspektivische Ansicht der Stange mit Prallplatte, Schraubenzugfeder und Diffusor im zusammengefügten Zustand,
- Fig. 5: eine perspektivische Ansicht der unteren Gehäusehäfte mit Stange, Prallplatte und Diffusor gemäß Fig. 4 in Einbaulage,
- Fig. 6: eine vergrößerte perspektivische Darstellung der Einzelheit X der Figur 2, und
- Fig. 7: eine perspektivische Darstellung des Halteelementes, des Sicherungselementes und des Mundstückes.

Nachfolgend sind gleiche Teile in den Zeichnungsfiguren mit denselben Bezugszeichen versehen.

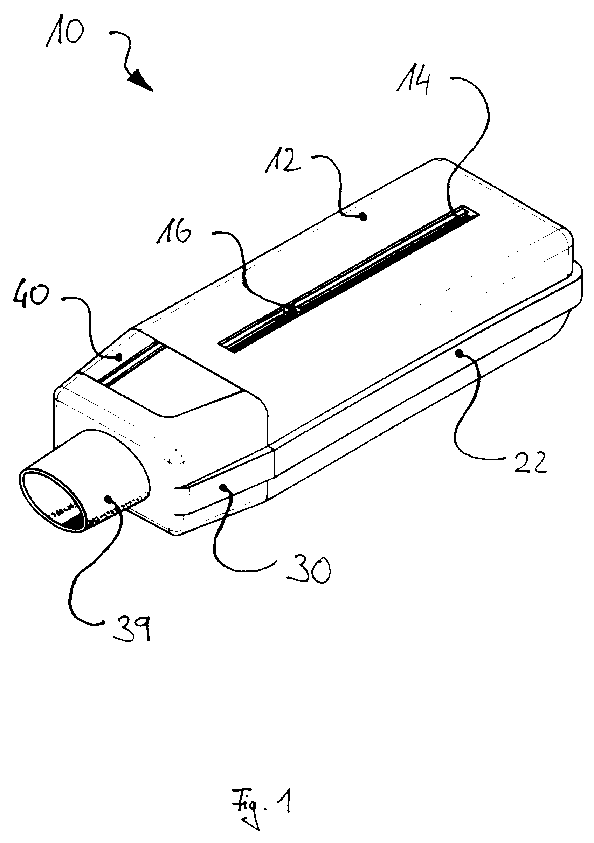

In Fig. 1 ist eine perspektivische Darstellung eines zusammengebauten Instrumentes 10 dargestellt. Das Gehäuse des Instrumentes 10 besteht aus zwei Gehäusehälften, einer oberen Gehäusehälfte 12 und einer unteren Gehäusehälfte 22. Die obere Gehäusehälfte 12 weist einen in Gehäuselängsrichtung verlaufenden Schlitz 14 auf, in dem ein entlang desselben bewegliches Anzeigeelement 16 angeordnet ist. Je nach Stärke des Luftstromes beim Ausatmen wird das Anzeigeelement 16 eine bestimmte Position im Schlitz 14 einnehmen. Eine oder zwei neben dem Schlitz aufgebrachte Skala bzw. Skalen (nicht dargestellt) erlaubt dann das Ablesen des Meßwertes in Einheiten wie beispielsweise l/min oder l/sec.. An seinem vorderen Ende weist das Instrument 10 eine Aufnahme 30 für ein Mundstück 39 auf, die mit einem Sicherungselement 40 am Gehäuse gehalten wird.

In Fig. 1A ist eine perspektivische Darstellung der beiden zusammengefügten Gehäusehälften des Instrumentes 10 dargestellt. Die obere Gehäusehälfte 12 ist hierbei teilweise aufgebrochen dargestellt, um den Vorsprung 18 deutlich darstellen zu können. Die obere Gehäusehälfte 12 weist den in Gehäuselängsrichtung verlaufenden Schlitz 14 sowie zwei Vorsprünge 18 auf, die als hervorstehende Nasen ausgebildet sind und von denen einer durch den Aufbruch des Gehäuses erkennbar ist. An ihrem vorderen Ende besitzt die obere Gehäusehälfte 12 einen Fortsatz 20. Die untere Gehäusehälfte 22 besitzt ebenfalls an ihrem vorderen Ende einen Fortsatz 26. Bei zusammengefügtem Gehäuse liegen die beiden Fortsätze 20 und 26, wie dargestellt, so aneinander an, daß sie die Lufteinlaßöffnung 21 umschließen. An ihrem hinteren Ende besitzt die untere Gehäusehälfte 22 zwei Luftauslaßöffnungen 24, in die die hervorstehenden Nasen 18 der obere Gehäusehälfte 12 bei zusammengefügten Gehäusehälften eingreifen.

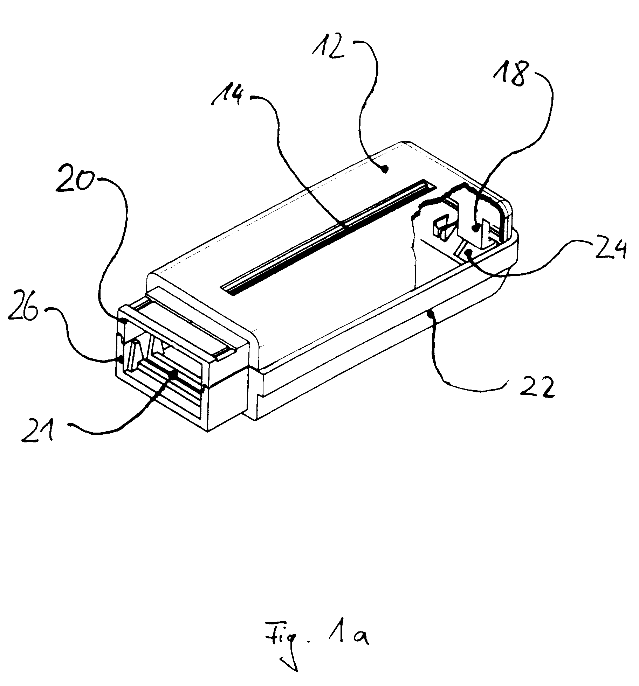

In Fig. 2 ist eine obere Gehäusehälfte 12 perspektivisch mit einem Aufbruch dargestellt, um eine der beiden an ihrem hinteren Ende angebrachten hervorstehenden Nasen 18 deutlich darstellen zu können.

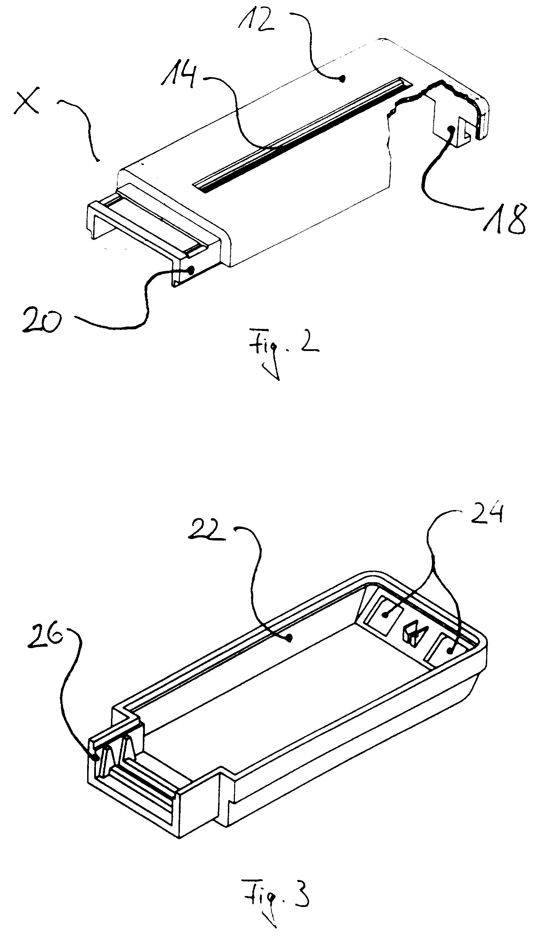

In Fig. 3 ist eine untere Gehäusehälfte 22 perspektivisch dargestellt. An dem hinteren Ende der Gehäusehälfte 22 sind die zwei Luftauslaßöffnungen 24 angebracht, in die bei zusammengefügten Gehäusehälften die hervorstehenden Nasen 18 der oberen Gehäusehälfte 12 eingreifen.

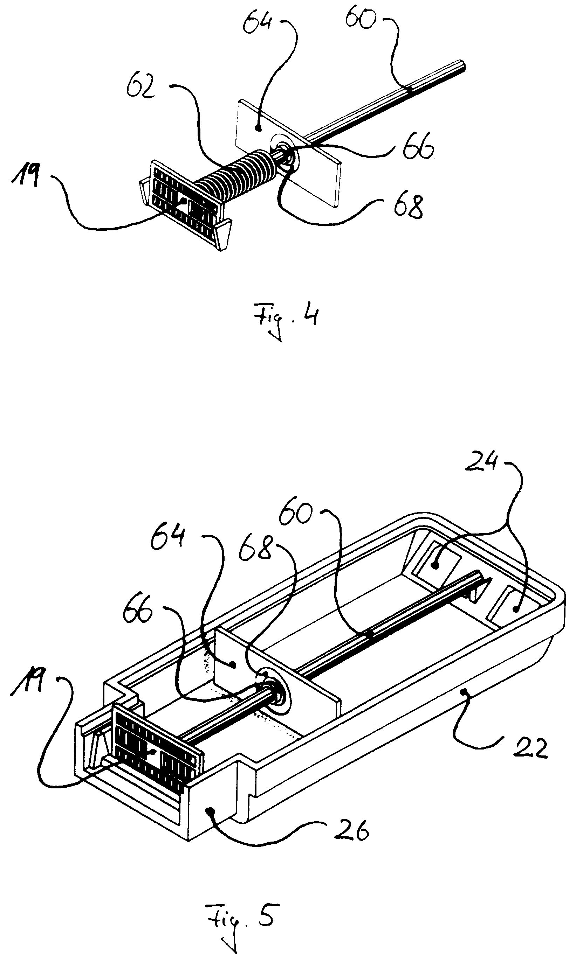

In Fig. 4 ist die Stange 60 mit Prallplatte 64, Schraubenzugfeder 62 und Diffusor 19 perspektivisch in Funktionsstellung dargestellt. Die Prallplatte 64 weist einen senkrecht zur Oberfläche stehenden durchbohrten Zapfen 66 auf, mit dem die Prallplatte 64 gleitend auf der Stange 60 gelagert ist. Ein Ende der Stange 60 ist in dem Diffusor 19 gelagert. Zwischen Diffusor 19 und Prallplatte 64 ist eine Schraubenzugfeder 22 um die Stange 60 herum angeordnet. Ein Ende der Schraubenfeder 62 weist einen in den Innenraum der Schraubenfeder gerichteten und die Mittelachse der Schraubenfeder schneidenden Schenkel auf, der mit einem Ende der Stange 60 zwischen dieser und dem Diffusor 9 eingeklemmt wird. Das andere Ende der Schraubenfeder 62 wird auf die Prallplatte 64 aufgeschraubt. Hierzu steht der Zapfen 66 auf der Prallplatte 64 von der Oberfläche in Richtung auf den Diffusor hervor und ist konisch ausgebildet. Um diesen Zapfen herum ist ein einmal um diesen herumführender Gewindegang 68 auf der Plattenoberfläche angeordnet, mit einer Steigung entsprechend der Steigung der Schraubenfeder 62. Das an der Prallplatte 64 zu befestigende Ende der Schraubenfeder 62 wird auf den Zapfen 66 aufgeschoben, bis es an dem Gewindegang 68 anliegt. Anschließend wird durch Drehen der Schraubenfeder 62 das Ende des die Schraubenfeder bildenden Drahtes durch eine im Gewindegang 68 angeordnete Bohrung hindurchgeführt, so daß sich durch weiteres Drehen die Schraubenfeder 62 in den Gewindegang 68 und damit in die Prallplatte 64 einschraubt.

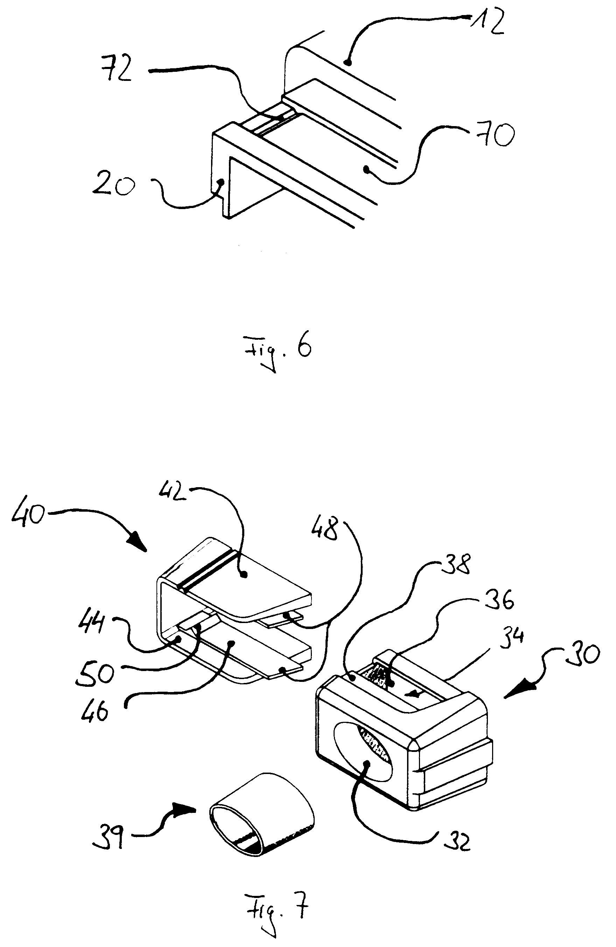

In Fig. 5 ist die untere Gehäusehälfte 22 mit der Stange 60, der Prallplatte 64 und dem Diffusor 19 in Einbaulage perspektivisch dargestellt. Der Diffusor 19, der ein Ende der Stange 60 lagert, wird in den Fortsatz 26 eingeschoben. Das zweite Ende der Stange 60 wird am hinteren Ende der Gehäusehälfte 22 gelagert.

In Fig. 6 ist eine vergrößerte perspektivische Darstellung der Ansicht X der Fig. 2 dargestellt. Diese Ansicht zeigt die im Fortsatz 20 der oberen Gehäusehälfte 12 angeordnete Nut 70. Diese Nut 70, die an ihren beiden Enden jeweils eine Ausnehmung 72 aufweist, ist auch an dem Fortsatz 26 der unteren Gehäusehälfte 22 in der bei zusammengefügtem Gehäuse nach außen weisenden Fläche angeordnet.

In Fig. 7 ist die als Halteelement ausgebildete Aufnahme 30 für ein Mundstück 39 zusammen mit dem Sicherungselement 40 und dem Mundstück 39 perspektivisch dargestellt. Die Aufnahme 30 besteht aus einem Hohlkörper, der an den beiden gegenüberliegenden Stirnseiten jeweils eine Öffnung 32, 34 aufweist. In die erste Öffnung 32 wird das Mundstück 39 eingeschoben, mit der zweiten Öffnung 34 wird die Aufnahme 30 auf die beiden aneinander anliegenden Fortsätze 20 und 26 der beiden Gehäusehälften 12, 22 aufgeschoben. An ihrer Mantelfläche weist die Aufnahme 30 zwei sich gegenüberliegende Öffnungen 36 auf, die innerhalb von Vertiefungen 38 angeordnet sind. In diese Vertiefungen 38 wird das klammerförmige Sicherungselement 40 eingeschoben. Das klammerförmige Sicherungselement 40 weist an den beiden Schenkeln 42 und 44 jeweils einen nach innen überstehenden Steg 46, der mit einer Nase 48 über die Enden der Schenkel 42 und 44 hinausragt. Im Bereich des nach innen gerichteten Endes weist der Steg 46 einen weiter nach innen hervorragenden Vorsprung 50 auf. Beim Einschieben des klammerförmigen Sicherungselementes 40 in die Vertiefungen 38 der Aufnahme 30 werden die Stege 46 durch die Öffnungen 36 hindurch in die Nuten 70 auf den Fortsätzen 20 und 26 der beiden Gehäusehälften 12, 22 eingeschoben. Dabei hintergreifen die Nasen 48 der Stege 46 die Mantelfläche der Aufnahme 30 von innen her und die Vorsprünge 50 auf den Stegen 46 rasten in die Ausnehmungen 72 in den Nuten 70, welche auf den Fortsätzen 20 und 26 angeordnet sind, ein.

Auf diese Weise wird eine formschlüssige, ohne große Kraftaufbringung manuell zu lösende Verbindung zwischen Sicherungselement 40, Halteelement 30 und den aneinander anliegenden Gehäusehälften 12 und 22 gebildet, so daß das Instrument 10 beispielsweise auch von Kindern und kranken oder alten Menschen für eine Reinigung leicht demontiert und anschließend wieder zusammengefügt werden kann. Aber auch bei der Herstellung kann das Gerät einfach und schnell und damit kostengünstig in der oben beschriebenen Weise montiert werden.

## Patentansprüche

1. Instrument zum Messen der maximalen Strömungsmenge während einer Ausatmung, bestehend aus
- einem Gehäuse aus zwei Gehäusehälften (12, 22), mit einer Lufteinlaßöffnung (21) und mindestens einer Luftauslaßöffnung (24) sowie einem in Gehäuselängsrichtung verlaufenden Schlitz (14) mit einem entlang dieses Schlitzes (14) beweglichen Anzeigeelement (16),
- einer im Gehäuse angeordneten Stange (60), die sich von der Einlaßöffnung (21) parallel zum Schlitz (14) in das Gehäuse hinein erstreckt und auf der eine Prallplatte (64) gleitend gelagert ist, die das entlang des Schlitzes (14) bewegliche Anzeigeelement (16) durch Mitnahme in einer Richtung bewegt,
- Federmitteln (62), die zwischen Gehäuse und Prallplatte (64) angeordnet sind und der Bewegung der Prallplatte (64) von der Einlaßöffnung (21) weg eine fortschreitend zunehmende Federkraft entgegensetzen, und
- einer am Gehäuse im Bereich der Lufteinlaßöffnung (21) angebrachten Aufnahme (30) für ein Mundstück,
dadurch gekennzeichnet, daß
- die erste Gehäusehälfte (12) mindestens einen Vorsprung (18) aufweist, der bei zusammengefügtem Gehäuse in eine Ausnehmung (24) in der zweiten Gehäusehälfte (22) eingreift,
- die erste (12) und die zweite Gehäusehälfte (22) jeweils mindestens einen Fortsatz (20, 26) aufweisen, die bei zusammengefügtem Gehäuse aneinander anliegen, und
- die beiden Fortsätze (20, 26) bei zusammengefügtem Gehäuse durch ein darüber geschobenes Halteelement (30) lösbar aneinander gehalten werden.

2. Instrument nach Anspruch 1, dadurch gekennzeichnet, daß die beiden Fortsätze (20, 26) an ihrer bei zusammengefügtem Gehäuse nach außen weisenden Fläche jeweils mindestens eine Nut (70) aufweisen und die Nuten (70) jeweils mit mindestens einer Ausnehmung (72) versehen sind.

3. Instrument nach Anspruch 1 oder 2, dadurch gekennzeichnet, daß die beiden Fortsätze (20, 26) bei zusammengefügtem Gehäuse die Lufteinlaßöffnung (21) umschließen.

4. Instrument nach Anspruch 3, dadurch gekennzeichnet, daß die Aufnahme (30) für das Mundstück (39) als Halteelement zum Aneinanderhalten der beiden Fortsätze (20, 26) ausgebildet ist.

5. Instrument nach Anspruch 4, dadurch gekennzeichnet, daß die Aufnahme (30) für das Mundstück (39) aus einem Hohlkörper besteht, der an den beiden sich gegenüberliegenden Stirnseiten jeweils eine Öffnung aufweist, wobei in die erste Öffnung (32) das Mundstück (39) und in die zweite Öffnung (34) die aneinander anliegenden Fortsätze (20, 26) der beiden Gehäusehälften (12, 22) eingeschoben wird, und der zwei sich gegenüberliegende Öffnungen (36) in der Mantelfläche aufweist, die innerhalb von Vertiefungen (38) angeordnet sind.

6. Instrument nach einem der Ansprüche 4 oder 5, dadurch gekennzeichnet, daß die Aufnahme (30) für das Mundstück (39) mit einem Sicherungselement (40) lösbar auf den beiden Fortsätzen (20, 26) arretiert ist.

7. Instrument nach Anspruch 6, dadurch gekennzeichnet, daß das Sicherungselement (40) klammerförmig ausgebildet ist und an den beiden Schenkeln (42, 44) nach innen überstehende Stege (46) aufweist, die mit Nasen (48) über die Enden der Schenkel (42, 44) hinausragen und an denen jeweils mindestens ein weiter nach innen hervorragender Vorsprung (50) angeordnet ist.

8. Instrument nach Anspruch 7, dadurch gekennzeichnet, daß das klammerförmige Sicherungselement (40) in die Vertiefungen (38) auf der Mantelfläche des Halteelementes (30) eingeschoben wird, wobei die Stege (46) des Sicherungselementes (40) durch die Öffnungen (36) in den Vertiefungen (38) der Mantelfläche hindurch in die Nuten (70) auf den Fortsätzen (20, 26) der beiden Gehäusehälften (12, 22) eingeschoben werden, die Nasen (48) an den Enden der Stege (46) die Mantelfläche des Halteelementes (30) von innen her hintergreifen und die an den Stegen (46) angeordneten Vorsprünge (50) in die Ausnehmungen (72) in den Nuten (70) einrasten, so daß eine formschlüssige manuell zu lösende Verbindung zwischen Sicherungselement (40), Halteelement (30) und Fortsätzen (20, 26) gebildet wird.

9. Instrument nach einem der vorhergehenden Ansprüche, dadurch gekennzeichnet, daß der mindestens eine Vorsprung (18) an der ersten Gehäusehälfte (12) als hervorstehende Nase ausgebildet ist, die bei zusammengefügtem Gehäuse in eine in der zweiten Gehäusehälfte (22) vorgesehene Luftauslaßöffnung (24) eingreift.

10. Instrument nach einem der vorhergehenden Ansprüche, dadurch gekennzeichnet, daß die Federmittel aus einer Schraubenzugfeder (62) bestehen, die um die im Gehäuse angeordnete Stange (60) herum angeordnet ist und an einem Ende einen in den Innenraum der Schraubenfeder (62) gerichteten und die Mittelachse der Schraubenfeder (62) schneidenden Schenkel aufweist, der mit einem Ende der Stange (60) zwischen dieser und einem in der Lufteinlaßöffnung (21) angeordneten Diffusor (19) festgeklemmt wird, und deren anderes Ende auf die Prallplatte (64) aufgeschraubt wird.

11. Instrument nach Anspruch 10, dadurch gekennzeichnet, daß die Prallplatte (64) einen senkrecht zur Oberfläche stehenden durchbohrten Zapfen (66), durch den die Stange (60) hindurch geschoben wird und auf den ein Ende der Schraubenfeder (62) aufgeschoben wird, sowie eine neben dem Zapfen (66) angeordnete, senkrecht durch die Prallplatte (64) hindurchführende Bohrung aufweist, an der ein einmal um den Zapfen (66) herumführender, auf der Plattenoberfläche angeordneter Gewindegang (68) mit einer Steigung entsprechend der Steigung der Schraubenfeder (62) beginnt.

## Claims

1. Instrument for measuring the maximum quantity of flow during expiration, comprising
- a housing of two housing halves (12, 22) having an air inlet opening (21) and at least one air outlet opening (24) as well as a slot (14) extending in the longitudinal direction of the housing and having a display element (16) which can be moved along this slot (14),
- a rod (60) arranged in the housing extending from the inlet opening (21) into the housing parallel to the slot (14) and on which an impact plate (64) is slidably mounted, which moves the display element (16) which can be moved along the slot (14) in one direction by slaving,
- spring means (62) which are arranged between housing and impact plate (64) and oppose the movement of the impact plate (64) away from the inlet opening (21) with a progressively increasing spring force, and
- a receiver (30) for a mouthpiece attached to the housing in the region of the air inlet opening (21),
characterised in that
- the first housing half (12) has at least one projection (18) which engages in a recess (24) in the second housing half (22) when the housing is assembled,
- the first (12) and the second housing half (22) have in each case at least one extension (20, 26) which rest against one another when the housing is assembled, and
- the two extensions (20, 26) are held releasably against one another when the housing is assembled by means of a retaining element (30) pushed thereover.

2. Instrument according to claim 1, characterised in that the two extensions (20, 26) at their surface facing outwards when the housing is assembled have in each case at least one groove (70) and the grooves (70) are provided in each case with at least one recess (72).

3. Instrument according to claim 1 or 2, characterised in that the two extensions (20, 26) surround the air inlet opening (21) when the housing is assembled.

4. Instrument according to claim 3, characterised in that the receiver (30) for the mouthpiece (39) is designed as a retaining element for holding the two extensions (20, 26) against one another.

5. Instrument according to claim 4, characterised in that the receiver (30) for the mouthpiece (39) comprises a hollow body, which has in each case an opening at the two opposing end-faces, wherein the mouthpiece (39) is pushed into the first opening (32) and the extensions (20, 26) resting against one another of the two housing halves (12, 22) are pushed into the second opening (34), and which has two opposing openings (36) in the outer surface which are arranged within depressions (38).

6. Instrument according to one of claims 4 or 5, characterised in that the receiver (30) for the mouthpiece (39) is locked releasably on the two extensions (20, 26) using a securing element (40).

7. Instrument according to claim 6, characterised in that the securing element (40) is designed like a clamp and has on the two limbs (42, 44) bars (46) projecting inwards which project beyond the ends of the limbs (42, 44) with noses (48) and on which is arranged in each case at least one projection (50) protruding further inwards.

8. Instrument according to claim 7, characterised in that the clamp-like securing element (40) is pushed into the depressions (38) on the outer surface of the retaining element (30), wherein the bars (46) of the securing element (40) are pushed through the openings (36) into the depressions (38) of the outer surface into the grooves (70) on the extensions (20, 26) of the two housing halves (12, 22), the noses (48) at the ends of the bars (46) engage the outer surface of the retaining element (30) from the inside and the projections (50) arranged on the bars (46) lock into the recesses (72) in the grooves (70), so that a positive connection to be released manually is formed between securing element (40), retaining element (30) and extensions (20, 26).

9. Instrument according to one of the preceding claims, characterised in that the at least one projection (18) on the first housing half (12) is designed as a protruding nose which engages in an air outlet opening (24) provided in the second housing half (22) when the housing is assembled.

10. Instrument according to one of the preceding claims, characterised in that the spring means comprise a helical extension spring (62) which is arranged around the rod (60) arranged in the housing and has at one end a limb directed into the interior of the helical spring (62) and cutting the central axis of the helical spring (62), which limb is firmly clamped by one end of the rod (60) between the latter and a diffusor (19) arranged in the air inlet opening (21), and the other end thereof is screwed onto the impact plate (64).

11. Instrument according to claim 10, characterised in that the impact plate (64) his a hollow journal (66) standing vertically to the surface, through which the rod (60) is pushed and is pushed onto one end of the helical spring (62), as well as a bore passing vertically through the impact plate (64) arranged next to the journal (66), on which bore starts a thread (68) arranged on the plate surface passing once around the journal (66) and having a pitch corresponding to the pitch of the helical spring (62).

## Revendications

1. Instrument pour mesurer la quantité d'écoulement maximale au cours d'une expiration, constitué par
- un boîtier constitué par deux moitiés de boîtier (12, 22) comportant une ouverture d'entrée (21) pour l'air et au moins une ouverture d'évacuation (24) pour l'air, ainsi qu'une fente (14) s'étendant dans la direction longitudinale du boîtier, un élément d'affichage (16) étant mobile le long de cette fente (14),
- une tige (60) disposée dans le boîtier, qui s'étend depuis l'ouverture d'entrée (21) parallèlement à la fente (14) pour pénétrer dans le boîtier et qui est montée en glissement sur une première plaque de rebondissement (64) qui déplace par entraînement dans une direction l'élément d'affichage (16) mobile le long de la fente (14),
- des éléments de ressort (62) qui sont disposés entre le boîtier et la plaque de rebondissement (64), et gui exercent une force de ressort antagoniste qui augmente progressivement à l'encontre du mouvement de la plaque de rebondissement (64) s'écartant de l'ouverture d'entrée (21), et
- un logement (30) pour un bec, disposé sur le boîtier dans la zone de l'ouverture d'entrée (21) pour l'air,
caractérisé en ce que
- la première moitié de boîtier (12) présente au moins une saillie (18) qui vient s'insérer, à l'état assemblé du boîtier, dans un évidement (24) pratiqué dans la seconde moitié de boîtier (22),
- la première moitié (12) et la seconde moitié (22) du boîtier présentent respectivement au moins un prolongement (20, 26) qui viennent s'appuyer l'un sur l'autre à l'état assemblé du boîtier, et
- les deux prolongements (20, 26) sont maintenus l'un contre l'autre de manière amovible, à l'état assemblé du boîtier, via un élément d'arrêt (30) appliqué par glissement sur ce dernier.

2. Instrument selon la revendication 1, caractérisé en ce que les deux prolongements (20, 26) présentent respectivement au moins une rainure (70) sur leur surface tournée vers l'extérieur à l'état assemblé du boîtier, les rainures (70) étant munies respectivement d'au moins un évidement (72).

3. Instrument selon la revendication 1 ou 2, caractérisé en ce que les deux prolongements (20, 26) entourent l'ouverture d'entrée (21) pour l'air, à l'état assemblé du boîtier.

4. Instrument selon la revendication 3, caractérisé en ce que le logement (30) destiné au bec (39) est réalisé sous forme d'élément d'arrêt pour maintenir l'un contre l'autre les deux prolongements (20, 26).

5. Instrument selon la revendication 4, caractérisé en ce que le logement (30) destiné au bec (39) est constitué d'un corps creux qui présente respectivement une ouverture sur ses deux côtés frontaux opposés l'un à l'autre, dans lequel on introduit par glissement le bec (39) dans la première ouverture (32) et on insère par glissement dans la seconde ouverture les deux prolongements (20, 26) des deux moitiés de boîtier (12, 22) s'appuyant l'un contre l'autre, et qui présente, dans la surface latérale, deux ouvertures (36) opposées l'une à l'autre, qui sont disposées à l'intérieur de renfoncements (38).

6. Instrument selon l'une quelconque des revendications 4 ou 5, caractérisé en ce que le logement (30) destiné au bec (39) est bloqué de manière amovible sur les deux prolongements (20, 26) à l'aide d'un élément de fixation (40).

7. Instrument selon la revendication 6, caractérisé en ce que l'élément de fixation (40) est réalisé en forme d'agrafe et présente, sur ses deux branches (42, 44), des nervures (46) faisant saillie vers l'intérieur, qui se prolongent avec des nez (48) au-delà des extrémités des branches (42, 44) et sur lesquelles est disposée respectivement au moins une saillie (50) s'étendant plus loin vers l'intérieur.

8. Instrument selon la revendication 7, caractérisé en ce que l'élément de fixation (40) en forme d'agrafe vient s'insérer par glissement dans les renfoncements (38) sur la surface latérale de l'élément d'arrêt (30), les nervures (46) de l'élément de fixation (40) venant s'insérer par glissement, à travers les ouvertures (36) pratiquées dans les renfoncements (38) de la surface latérale, dans les rainures (70) disposées sur les prolongements (20, 26) des deux moitiés de boîtier (12, 22), les nez (48) aux extrémités des nervures (46) venant s'appliquer par l'arrière depuis l'intérieur contre la surface latérale de l'élément d'arrêt (30) et les saillies (50) disposées sur les nervures (46) venant s'encliqueter dans les évidements (72) pratiqués dans les rainures (70), si bien que l'on obtient une liaison mécanique qui doit être libérée à la main, entre l'élément de fixation (40), l'élément d'arrêt (30) et les prolongements (20, 26).

9. Instrument selon l'une quelconque des revendications précédentes, caractérisé en ce que la ou les saillies (18) sur la première moitié de boîtier (12) est ou sont réalisées sous la forme d'un nez faisant saillie vers l'avant, qui vient s'insérer, à l'état assemblé du boîtier, dans une ouverture d'évacuation (24) pour l'air prévue dans la deuxième moitié de boîtier (22).

10. Instrument selon l'une quelconque des revendications précédentes, caractérisé en ce que les éléments de ressort sont constitués par un ressort de traction à vis (62) qui est disposé autour de la tige (60) disposée dans le boîtier et qui présente, à une de ses extrémités, une branche tournée vers l'espace interne du ressort à vis (62) et qui coupe l'axe médian du ressort à vis (62), ladite branche étant serrée avec une extrémité de la tige (60) entre cette dernière et un diffuseur (19) disposé dans l'ouverture d'entrée (21) pour l'air et dont l'autre extrémité est vissée sur la plaque de rebondissement (64).

11. Instrument selon la revendication 10, caractérisé en ce que la plaque de rebondissement (64) présente un tourillon (66) perforé, disposé perpendiculairement à la surface, à travers lequel vient s'insérer la tige (60) par glissement et sur lequel glisse une extrémité du ressort à vis (62), ainsi qu'un alésage disposé à côté du tourillon (66) et traversant la plaque de rebondissement (64) à la verticale, contre lequel commence un pas de vis (68) disposé sur la surface de la plaque, tournant une fois autour du tourillon (66), avec un pas correspondant au pas du ressort à vis (62).
